# EUROPÄISCHE PATENTSCHRIFT

(11) **EP 1 532 091 B1**
(45) Veröffentlichungstag und Bekanntmachung des Hinweises auf die Patenterteilung: **11.04.2007**
(21) Anmeldenummer: 03792383.6
(22) Anmeldetag: 20.08.2003
(51) Int. Cl.: C07C 29/17, C07C 29/20, C07C 29/56, C07C 35/12

(54) **VERFAHREN ZUR HERSTELLUNG VON MENTHOL**
METHOD FOR PRODUCING MENTHOL
PROCEDE POUR LA PRODUCTION DE MENTHOL

(30) Priorität: 22.08.2002 DE 10239274
(43) Veröffentlichungstag der Anmeldung: 25.05.2005
(73) Patentinhaber: Symrise GmbH & Co. KG, 37603 Holzminden (DE)
(72) Erfinder: KUHN, Walter, 37603 Holzminden (DE); FUNK, Hans-Ulrich, 37697 Lauenförde (DE); SENFT, Gerhard, 37603 Holzminden (DE); KÖRBER, Konrad, Alfred, 37603 Holzminden (DE)
(74) Vertreter: Eisenführ, Speiser & Partner
(86) Internationale Anmeldenummer: PCT/EP2003/009193
(87) Internationale Veröffentlichungsnummer: WO 2004/018398

(56) Entgegenhaltungen:
- EP-A- 0 743 295
- GB-A- 1 503 723
- DATABASE WPI Section Ch, Week 196800 Derwent Publications Ltd., London, GB; Class B00, AN 1966-17676F XP002263812 & SU 167 842 A (KOLOGRILOVA N E)
- ALLAKHVERDIEV A I ET AL: "LIQUID-PHASE STEREOSELECTIVE THYMOL HYDROGENATION OVER SUPPORTED NICKEL CATALYSTS" CATALYSIS LETTERS, BALTZER, SCIENTIFIC PUBL, BASEL, CH, Bd. 29, Nr. 1/2, 1. November 1994 (1994-11-01), Seiten 57-67, XP000579755 ISSN: 1011-372X

## Beschreibung

Die vorliegende Erfindung betrifft ein Verfahren zur Herstellung von Menthol durch katalytische Hydrierung von Verbindungen, die das Kohlenstoffgerüst des Menthans mit wenigstens einer Doppelbindung besitzen und in 3-Stellung durch Sauerstoff substituiert sind und/oder katalytische Umlagerung von Stereoisomeren des Menthols in Gegenwart von Wasserstoff und dotierten Nickel-Katalysatoren.

Von den natürlich vorkommenden cyclischen Terpenalkoholen ist 1-Menthol ein besonders wichtiger Vertreter. L-Menthol hat einen einzigartigen erfrischenden Geschmack, einen minzigen Geruch und eine stark wirkende Kühlung auf Haut und Schleimhaut. Es wird bei der Mundpflege, in kosmetischen und pharmazeutischen Präparaten, im Tabak und in Süßwaren verwendet.

Die 8 stereoisomeren Menthole (je zwei Enantiomere von Menthol, Neomenthol, Isomenthol und Neoisomenthol, siehe auch K. Bauer, D. Garbe und H. Surburg, Common Fragrance and Flavor Materials, 4^{th} Ed., Wiley-VCH, Weinheim 2001, S.52-55) unterscheiden sich in Bezug auf ihre organoleptischen Eigenschaften. So hat 1-Menthol einen charakteristischen Pfefferminzgeruch und die schon erwähnte erfrischende Wirkung; es ist deshalb das wertvollste Menthol-Stereoisomer. Man ist daher bestrebt, die Hydrierung so zu führen, dass möglichst viel Menthol entsteht bzw. Stereoisomere des Menthols, wie sie beispielsweise bei der Thymol-Hydrierung anfallen, möglichst effektiv in Menthol umzulagern.

Die katalytische Hydrierung von Verbindungen, die das Kohlenstoffgerüst des Menthans mit wenigstens einer C=C-Doppelbindung besitzen und in 3-Stellung durch Sauerstoff substituiert sind und/oder die katalytische Umlagerung von Stereoisomeren des Menthols in Gegenwart von Wasserstoff führt zu racemischem Menthol.

In Liebigs Annalen 1960, 637, 1 wird die Isomerisierung von hydriertem Thymol mittels Raney Nickel in Methanol bei 200°C bis 250°C beschrieben, wobei das Produktgemisch einen Gehalt an d,l-Menthol von 57,1 % aufweist.

In EP-A 563 611 und DE-A 197 18 116 wird Menthol durch katalytische Hydrierung und/oder katalytische Umlagerung in Gegenwart von Wasserstoff und EdelmetallKatalysatoren hergestellt.

In DE-OS 2 314 813 wird d,l-Menthol unter Verwendung eines Co-Mn-Festbettkatalysators erhalten. In einem Vergleichsbeispiel wurde ein Gemisch, das im Wesentlichen aus Thymol und d-Neomenthol bestand, in Gegenwart von Raney-Nickel bei 210°C und 280 bar Wasserstoffdruck in d,1-Menthol überführt. Das Reaktionsprodukt enthielt 56,1 % Menthol, daneben auch 4,8 % Kohlenwasserstoffe.

In GB 1 503 723 wird die stereoselektive Hydrierung von monozyklischen Terpen-Allylalkoholen und ungesättigten Verbindungen. Als besonders anwendbar wird die stereoselektive Hydrierung von Terpenen mit monozyklischen Allylalkoholen und hier insbesondere von d-trans-Piperitol zu d-Isomenthol beschrieben.

Unter wirtschaftlichen Gesichtspunkten liefern die bislang bekannten katalytischen Hydrier- und/oder Umlagerungsverfahren das Menthol nur mit unbefriedigender chemischer Ausbeute oder Selektivität (z.B. zu geringer Menthol-Gehalt, Bildung von Kohlenwasserstoffen als unerwünschte Nebenprodukte), unzureichender Raum-Zeit-Ausbeute (z.B. lange Reaktionszeiten) und/oder benötigen teure EdelmetallKatalysatoren.

Die Aufgabe der vorliegenden Erfindung bestand darin, ein verbessertes Verfahren zur Herstellung von Menthol mittels katalytischer Hydrierung und/oder katalytischer Umlagerung zu finden, insbesondere unter wirtschaftlichen Aspekten und im industriellen Maßstab.

Die vorliegende Erfindung betrifft daher ein Verfahren zur Herstellung von Menthol durch katalytische Hydrierung von Ausgangsmaterialien, die das Kohlenstoffgerüst des Menthans mit wenigstens einer Doppelbindung besitzen und in 3-Stellung durch Sauerstoff substituiert sind und/oder katalytische Umlagerung von Stereoisomeren des Menthols in Gegenwart von Wasserstoff, dadurch gekennzeichnet, dass die Reaktion in Gegenwart eines mit Eisen und/oder Chrom dotierten Nickel-Katalysators und bei einer Temperatur im Bereich 80-230°C und Wasserstoffdrücken im Bereich 1-200 bar abs. durchgeführt wird, wobei der dotierte Nickel-Katalysator im trockenen Zustand einen Eisenanteil von 0,1-20 Gew.-%, einen Chromanteil von 0,1-20 Gew.-%, einen Nickelanteil von 60-95 Gew.-% und einen Aluminiumanteil von 1-20 Gew.-% aufweist.

Unter einem Nickel-Katalysator wird in der vorliegenden Erfindung ein Katalysator mit einem Nickelgehalt von mindestens 50 Gew.-%, bezogen auf den trockenen Katalysator, verstanden.

Als Ausgangsmaterialien eignen sich für die erfindungsgemäße Reaktion Verbindungen, die sich vom p-Menthan (4-Isopropyl-1-methyl-cyclohexan) ableiten und in 3-Position durch Sauerstoff substituiert sind, wie beispielsweise Menthon, Menthenon, Isomenthon, Neomenthol, Isomenthol, Neoisomenthol, Isopulegol, Neo-Isopulegol, Iso-Isopulegol, Neoiso-Isopulegol, Piperiton, Piperitol, Piperitenol oder Isopiperitenol. Diese Verbindungen können entantiomerenrein, enantiomerenangereichert oder racemisch eingesetzt werden. Insbesondere auch Thymol, d-Menthol und 1-Menthol können verwendet werden. Diese Verbindungen können sowohl einzeln als auch in beliebigen Gemischen als Ausgangsmaterial verwendet werden.

Für das erfindungsgemäße Verfahren kann der Katalysator im trockenen oder feuchten Zustand (Wassergehalt bis zu 60 Gew.-%) verwendet werden.

Die im Folgenden angegebenen Metallanteile beziehen sich auf das Gewicht des trockenen Katalysators.

Erfindungsgemäß bevorzugt ist der Einsatz von Fe- und Cr-dotierten Raney-Nickel-Katalysatoren (Raney-Nickel-Eisen-Chrom). Besonders vorteilhaft sind dabei Katalysatoren enthaltend bzw. bestehend aus:
Fe: 0,1-20 Gew.-%, besonders bevorzugt 1-10 Gew.-%;
Cr: 0,1-20 Gew.-%, besonders bevorzugt 1-10 Gew.-%,
Ni: 60-95 Gew.-%, besonders bevorzugt 80-93 Gew.-%,
A1: 1-20 Gew.-%, besonders bevorzugt 3-10 Gew.-%.

Für das erfindungsgemäße Verfahren beträgt das Gewichtsverhältnis des eingesetzten trockenen Nickel-Katalysators zu Ausgangsmaterial 0,001 - 0,1 : 1, bevorzugt 0,005 - 0,05 : 1, besonders bevorzugt 0,01 - 0,03 : 1.

Im Falle eines kontinuierlichen Verfahrens bezieht sich dieses Gewichtsverhältnis auf die Gesamtmenge des in die Hydrierung eingebrachten Ausgangsmaterials, bezogen auf die Gesamtmenge des eingesetzten Nickel-Katalysators.

Das Verfahren wird erfindungsgemäß bei 80-230°C, bevorzugt bei 120-210°C insbesondere bevorzugt bei 150-190°C durchgeführt.

Das erfindungsgemäße Verfahren wird mit Wasserstoff durchgeführt, die Wasserstoffdrücke liegen üblicherweise im Bereich 1 bis 200 bar abs.

Bevorzugt sind bei einer diskontinuierlichen Reaktionsführung Wasserstoffdrücke im Bereich 1 bis 100 bar abs., besonders im Bereich 3 bis 50 bar abs., insbesondere im Bereich 5 bis 25 bar abs..
Bevorzugt sind bei einer kontinuierlichen Reaktionsführung Wasserstoffdrücke im Bereich 50 bis 200 bar abs., insbesondere im Bereich 100 bis 200 bar abs..

Die Reaktionszeit liegt üblicherweise im Bereich 1 bis 100 Stunden, bevorzugt im Bereich 10 bis 70 Stunden, besonders bevorzugt 20 bis 55 Stunden.

Das Verfahren kann kontinuierlich, semi-kontinuierlich und diskontinuierlich durchgeführt werden.

Im Falle einer diskontinuierlichen Reaktionsführung (Batch) wird der dotierte Nickel-Katalysator bevorzugt als Pulver, vorteilhafterweise ohne Trägermaterial, eingesetzt.

Im Falle einer kontinuierlichen Reaktionsführung kann es vorteilhaft sein, den dotierten Nickel-Katalysator als Formkörper oder Hohlkörper zu verwenden. Es können Körper jeglicher Gestalt eingesetzt werden, wie beispielsweise Hohlstränge, Extrudate, Pellets, Strangpresslinge, Wirbelstränge, Sättel, Ringe, Hohlkugeln, Kugeln, Hohlzylinder, Zylinder, Würfel, Tabletten, Kegel und dergleichen. Es ist dabei vorteilhaft kein Trägermaterial zu verwenden, sondern den dotierten Nickel-Katalysator als solchen als Körper auszugestalten.

Ebenfalls vorteilhaft ist bei der kontinuierlichen Fahrweise die Reaktionsführung in einem Festbettreaktor, bevorzugt in einem Rieselbettreaktor.

Die Tabelle 1 zeigt die Ergebnisse der Umsetzung (Reaktionsbedingungen: 20 bar Wasserstoffdruck, 175°C, kein Verdünnungsmittel, 1 Gew.-% trockener Katalysator, bezogen auf Thymol, Wassergehalt des Katalysators: 50 Gew.-%) von Thymol an verschiedenen Nickel-Katalysatoren nach 4 Stunden.

Der Katalysator Ra-Ni-Cr-Fe ist erfindungsgemäß, wohingegen die Katalysatoren Ra-Ni, Ra-Ni-Cr und Ra-Ni-Fe nicht erfindungsgemäße Vergleichsbeispiele sind.

**Tabelle 1 :**

| **Katalysator** | **Thymol GC-%** | **Menthol GC-%** | **Isomenthol GC-%** | **Neomenthol GC-%** | **Neoisomenthol GC-%** |
|---|---|---|---|---|---|
| Ra-Ni | 42,1 | 1,9 | 12,4 | 4,1 | 19,7 |
| Ra-Ni-Cr | 19,3 | 11,8 | 22,8 | 13,1 | 21,5 |
| Ra-Ni-Fe | 30,8 | 4,3 | 37,7 | 7,4 | 14,5 |
| Ra-Ni-Cr-Fe | 0,1 | 27,3 | 38,5 | 18,5 | 11,6 |

Die restlichen GC-Prozente entfallen im Wesentlichen auf Menthon und Isomenthon. Die eingesetzten Katalysatoren hatten folgende Zusammensetzungen, die Angaben beziehen sich auf das Gewicht des trockenen Katalysators (Bezugsquelle: Degussa AG):

| | |
|---|---|
| Ra-Ni-Fe: | 82 % Nickel; 7 % Aluminium; 11 % Eisen |
| Ra-Ni-Cr: | 88 % Nickel; 9 % Aluminium; 3 % Chrom |
| Ra-Ni-Cr-Fe: | 90 % Nickel; 7 % Aluminium; 1 % Eisen; 2 % Chrom |

Das erfindungsgemäße Verfahren kann unter Verwendung von Verdünnungsmitteln oder Verdünnungsmittelgemischen durchgeführt werden. Geeignet sind beispielsweise Alkohole, wässrige Alkohole, cyclische oder acyclische Ether und gesättigte cyclische oder acyclische Kohlenwasserstoffe. Üblicherweise können Verdünnungsmittel wie Methanol, Ethanol, Isopropanol, n-Propänol, Isobutanol, n-Butanol, sek.-Butanol, Tetrahydrofuran, Dibutylether, Ethylenglykoldimethylether, Pentan, Hexan, Heptan, Octan, Isooctan, Cyclopentan, Cyclohexan, Methylcyclohexan oder Cyclooctan verwendet werden.

Das erfindungsgemäße Verfahren wird bevorzugt im Wesentlichen verdünnungsmittelfrei durchgeführt. Hierunter wird ein Anteil von weniger als 5 Gew.-%, bevorzugt von weniger als 2 Gew.-%, an Verdünnungsmittel bezogen auf das Ausgangsmaterial verstanden.

Für das erfindungsgemäße Verfahren können zusätzlich basische Zusätze der Gruppe der Alkalihydroxide, der Erdalkalihydroxide, der Alkalialkoholate, der Erdalkalialkoholate und der Amine verwendet werden.

Bei den Alkalihydroxiden sind Natriumhydroxid und Kaliumhydroxid bevorzugt. Die Alkali- und Erdalkalihydroxide können in purum oder als wässrige oder alkoholische Lösung eingesetzt werden.

Die Alkoholate umfassen bevorzugt 1 bis 4 Kohlenstoffatome und sind vorzugsweise gewählt aus der Gruppe Methanolat, Ethanolat, Isopropanolat und tert.-Butanolat, besonders bevorzugt sind Methanolat und Ethanolat. Ganz besonders bevorzugte Alkalialkoholate sind Natrium-Methanolat und Natrium-Ethanolat.

Die Alkoholate können in purum oder in Alkohol gelöst eingesetzt werden, dabei bevorzugt in dem dem Alkoholat entsprechenden Alkohol. Der Anteil des Alkoholats in alkoholischer Lösung liegt üblicherweise im Bereich 10-70 Gew.-%, bevorzugt bei 15-50 Gew.-%.

Bei den Aminen sind Triethylamin, Tributylamin, Ethanolamin und Dimethylbenzylamin bevorzugt.

Für das erfindungsgemäße Verfahren beträgt das Gewichtsverhältnis des basischen Zusatzes (einzeln bzw. Summe der basischen Zusätze) zu Ausgangsmaterial üblicherweise 0,000001 - 0,1 : 1, bevorzugt 0,00001 - 0,05 : 1, insbesondere bevorzugt 0,001 - 0,02 : 1. Das erfindungsgemäße Verfahren kann beispielsweise folgendermaßen durchgeführt werden:

In einem Druckbehälter werden Ausgangsmaterial, Nickel-Katalysator sowie gegebenenfalls Verdünnungsmittel und basischer Zusatz vorgelegt. Es erfolgt dann bei der gewählten Temperatur und dem gewählten Wasserstoffdruck die Hydrierung bzw. Umlagerung. Nach beendeter Reaktion wird das rohe Menthol durch Entfernen des Katalysators (z.B. durch Filtration, Dekantierung, Zentrifugierung) und gegebenenfalls durch Entfernen des basischen Zusatzes über Waschvorgänge, erhalten. Im Anschluss wird das rohe Menthol üblicherweise weiter aufgereinigt, beispielsweise durch Destillation oder Kristallisation.

Bei Einsatz von enantiomerenreinen bzw. enantiomerenangereicherten Ausgangsmaterialien in die Umlagerungsreaktion findet unter den Reaktionsbedingungen Racemisierung oder Teilracemisierung statt. Die bei dem erfindungsgemäßen Verfahren erfolgenden Hydrierungen, Racemisierungen sowie Isomerisierungen führen überraschenderweise in hohem Maße zur Bildung von Menthol unter Verwendung eines preiswerten Nickel-Katalysators, der nicht auf Edelmetallen basiert noch Edelmetall-Dotierungen benötigt. Insbesondere überraschend ist, dass mit dem erfindungsgemäßen Verfahren ein weitestgehend Kohlenwasserstoff-freies Produkt (typischerweise <0,5 Gew.%) erhalten wird.

Insbesondere die milden Reaktionsbedingungen stellen im industriellen Maßstab eine wesentliche Verbesserung dar.

Folgende Beispiele erläutern die Erfindung:

Sofern nicht anders angegeben beziehen sich alle Angaben auf das Gewicht.

### Beispiel 1: Herstellung von d,l-Menthol ex Thymol

In einem 5 1 Rührautoklaven mit Begasungsrührer wurden 996 g Thymol (Reinheit: 99,8 %) und 20 g Raney-Nickel-Eisen-Chrom (Zusammensetzung des trockenen Katalysators: 92 % Nickel, 5 % Aluminium, 2 % Chrom, 1 % Eisen; Wassergehalt 50 %; Form des Katalysators: Pulver; Quelle: Degussa AG) vorgelegt. Es wurde 24 Stunden bei 175°C und 1 Stunde bei 100°C hydriert. Der Wasserstoffdruck lag bei 20 bar. Nach Filtration wurden 1028 g eines Menthol-Isomerengemisches folgender Zusammensetzung (Summe der Menthole 99,5 %) erhalten:

| | |
|---|---|
| 60,3 % | Menthol |
| 25,8 % | Neomenthol |
| 11,7 % | Isomenthol |
| 1,7 % | Neoisomenthol |

Das erhaltene Menthol-Isomerengemisch konnte ohne organischen Rückstand bis 100°C Sumpftemperatur und 1 mbar destilliert werden.

### Beispiel 2: Herstellung von Menthol ex Thymol

Die Reaktionsführung entsprach Beispiel 1, lediglich die Reaktionstemperatur wurde auf 165°C abgesenkt. Das erhaltene Menthol-Isomerengemisch hatte folgende Zusammensetzung (Summe der Menthole 99,7 %):

| | |
|---|---|
| 60,6 % | Menthol |
| 26,3 % | Neomenthol |
| 11,3 % | Isomenthol |
| 1,5 | % Neoisomenthol |

### Beispiel 3: Herstellung von Menthol ex Thymol

Die Reaktionsführung entsprach Beispiel 1, lediglich die Reaktionstemperatur wurde auf 185°C geändert. Das erhaltene Menthol-Isomerengemisch hatte folgende Zusaanmensetzung (Summe der Menthole 97,7 %):

| | |
|---|---|
| 57,6 % | Menthol |
| 26,5 % | Neomenthol |
| 11,7 % | Isomenthol |
| 1,9 % | Neoisomenthol |

### Beispiel 4: Herstellung von Menthol ex Menthon-Isomenthon-Gemisch

In einem 5 1 Rührautoklaven mit Begasungsrührer wurden 996 g Menthon-Isomenthon-Gemisch (Reinheit: 99,9 %; Menthon-Gehalt 85,8 %, Isomenthon-Gehalt 14,2 %) und 20 g Raney-Nickel-Eisen-Chrom (Zusammensetzung: siehe Beispiel 1) vorgelegt. Es wurde 25 Stunden bei 165°C und 1 Stunde bei 100°C hydriert. Der Wasserstoffdruck lag bei 18 bar. Nach Filtration wurden 1003 g eines Menthol-Isomerengemisches mit folgender Zusammensetzung (Summe der Menthole 99,8 %) erhalten:

| | |
|---|---|
| 61,9 % | Menthol, |
| 25,5 % | Neomenthol |
| 11,1 % | Isomenthol |
| 1,3 % | Neoisomenthol |

Das erhaltene Menthol-Isomerengemisch konnte ohne organischen Rückstand bis 100°C Sumpftemperatur und 1 mbar destilliert werden.

### Beispiel 5: Herstellung von Menthol ex Menthol-Isomerengemisch

In einem 5 1 Rührautoklaven mit Begasungsrührer wurden 992 g eines Menthol-Isomerengemisches folgender Zusammensetzung (Summe der Menthole 95,3 %):

| | |
|---|---|
| 9,2 % | Menthol |
| 81,9 % | Neomenthol |
| 0,1 % | Isomenthol |
| 4,1 % | Neoisomenthol |

und 20 g Raney-Nickel-Eisen-Chrom (Zusammensetzung: siehe Beispiel 1) vorgelegt. Es wurde 20 Stunden bei 175°C und 1 Stunde bei 100°C isomerisiert. Der Wasserstoffdruck lag bei 18 bar. Nach Filtration wurden 990 g eines Menthol-Isomerengemisches folgender Zusammensetzung (Summe der Menthole 94,2 %) erhalten:

| | |
|---|---|
| 56,8 % | Menthol |
| 25,0 % | Neomenthol |
| 10,8 % | Isomenthol und |
| 1,6 % | Neoisomenthol |

Das erhaltene Menthol-Isomerengemisch konnte ohne organischen Rückstand bis 100°C Sumpftemperatur und 1 mbar Vakuum destilliert werden.

### Beispiel 6: Herstellung von Menthol ex Isopulegol

In einem 5 1 Rührautoklaven mit Begasungsrührer werden 993 g Isopulegol der Zusammensetzung (Summe der Isopulegole 97,6 %)

| | |
|---|---|
| 70,1 % | Isopulegol, |
| 18,1% | Neo-Isopulegol |
| 6,8 % | Iso-Isopulegol und |
| 2,6 % | Neoiso-Isopulegol |

und 20 g Raney-Nickel-Eisen-Chrom (Zusammensetzung: siehe Beispiel 1) vorgelegt. Es wurde insgesamt 20 Stunden bei 175°C und 1 Stunde bei 100°C hydriert. Der Wasserstoffdruck betrug 18 bar. Nach Filtration wurden 1019 g eines Menthol-Isomerengemisches folgender Zusammensetzung (Summe der Menthole 97,0 %) erhalten:

| | |
|---|---|
| 61,4 % | Menthol |
| 25,5 % | Neomenthol |
| 8,8 % | Isomenthol |
| 1,3 % | Neoisomenthol |

Das erhaltene Menthol-Isomerengemisch konnte ohne organischen Rückstand bis 100°C Sumpftemperatur und 1 mbar destilliert werden.

### Beispiel 7: Herstellung von d,l-Menthol ex d-Menthol

In einem 5 l Rührautoklaven mit Begasungsrührer wurden 985 g d-Menthol (Reinheit: 99,9 %; d-Menthol >95 %, 1-Menthol <5 %) und 20 g Raney-Nickel-Eisen-Chrom (Zusammensetzung: siehe Beispiel 1) vorgelegt. Die Reaktionsmischung wurde 50 Stunden bei 175°C und 2 Stunden bei 100°C bei einem Wasserstoffdruck von 18 bar gehalten. Nach Filtration wurden 976 g eines Menthol-Isomerengemisches folgender Zusammensetzung (Summe der Menthole 99,5 %) erhalten:

| | |
|---|---|
| 60,6 % | d,l-Menthol (d-Menthol 49 %, 1-Menthol 51 %) |
| 25,8 % | d,l-Neomenthol (d-Neomenthol 46 %,1-Neomenthol 54 %) |
| 11,5 % | d,l-Isomenthol (d-Isomenthol 46 %,1-Isomenthol 54 %) |
| 1,6 % | d,l-Neoisomenthol; |

Das erhaltene Menthol-Isomerengemisch konnte ohne organischen Rückstand bis 100°C Sumpftemperatur und 1 mbar Vakuum destilliert werden.

Die Zusammensetzung wurde mittels GC bestimmt. GC-Säule für die EnantiomerenTrennung des Menthol-Isomerengemisches: Permethyllertes-β-Cyclodextrin von J&W; Bedingungen: Trägergas: Helium, 1 bar; TemperaturProgramm: 80 - 180°C, Heizrate 2°C/min.

## Patentansprüche

1. Verfahren zur Herstellung von Menthol durch katalytische Hydrierung von Ausgangsmaterialien, die das Kohlenstoffgerüst des Menthans mit wenigstens einer Doppelbindung besitzen und in 3-Stellung durch Sauerstoff substituiert sind und/oder katalytische Umlagerung von Stereoisomeren des Menthols in Gegenwart von Wasserstoff, **dadurch gekennzeichnet, dass** die Reaktion in Gegenwart eines mit Eisen und/oder Chrom dotierten Nickel-Katalysators und bei einer Temperatur im Bereich 80-230°C und Wasserstoffdrücken im Bereich 1-200 bar abs. durchgeführt wird, wobei der dotierte Nickel-Katalysator im trockenen Zustand einen Eisenanteil von 0,1-20 Gew.-%, einen Chromanteil von 0,1-20 Gew.-%, einen Nickelanteil von 60-95 Gew.-% und einen Aluminiumanteil von 1-20 Gew.-% aufweist.

2. Verfahren nach Anspruch 1, **dadurch gekennzeichnet, dass** der Nickel-Katalysator im trockenen Zustand einen Eisenanteil von 0,1-10 Gew.%, einen Chromanteil von 0,1-10 Gew.-%, einen Nickelanteil von 80-93 Gew.% und einen Aluminiumanteil von 3-10 Gew.-% aufweist.

3. Verfahren nach mindestens einem der Ansprüche 1 bis 2, **dadurch gekennzeichnet, dass** das Verfahren im Wesentlichen verdünnungsmittelfrei durchgeführt wird.

4. Verfahren nach mindestens einem der Ansprüche 1 bis 3, **dadurch gekennzeichnet, dass** das Gewichtsverhältnis des trockenen Nickel-Katalysators zu Ausgangsmaterialien im Bereich 0,001 - 0,1 : 1 liegt.

5. Verfahren nach mindestens einem der Ansprüche 1 bis 4, **dadurch gekennzeichnet, dass** die Reaktionstemperatur zwischen 120 und 210°C liegt.

6. Verfahren nach mindestens einem der Ansprüche 1 bis 5, **dadurch gekennzeichnet, dass** das Verfahren diskontinuierlich durchgeführt wird.

7. Verfahren nach Anspruch 6, **dadurch gekennzeichnet, dass** der Wasserstoffdruck bei 3 bis 50 bar abs. liegt.

## Claims

1. Method for the manufacture of menthol by catalytic hydration of source materials which possess the carbon structure of the menthane with at least one double bond and are substituted in the 3-position by oxygen, and/or catalytic rearrangement of stereo isomers of the menthol in the presence of hydrogen, **characterised in that** the reaction is carried out in the presence of a nickel catalyst doped with iron and/or chromium and at a temperature in the range 80-230°C and hydrogen pressures in the range 1-200 bar absolute, the doped nickel catalyst in the dry state having an iron portion of 0.1-20% by weight, a chromium portion of 0.1-20% by weight, a nickel portion of 60-95% by weight, and an aluminium portion of 1-20% by weight.

2. Method according to Claim 1, **characterised in that** the nickel catalyst in the dry state has an iron portion of 0.1-10% by weight, a chromium portion of 0.1-10% by weight, a nickel portion of 80-93% by weight, and an aluminium portion of 3-10% by weight.

3. Method according to at least one of Claims 1 to 2, **characterised in that** the method is carried out essentially free of solvents.

4. Method according to at least one of Claims 1 to 3, **characterised in that** the weight ratio of the dry nickel catalyst to the source materials is in the range from 0.001-0.1 : 1.

5. Method according to at least one of Claims 1 to 4, **characterised in that** the reaction temperature is between 120 and 210°C.

6. Method according to at least one of Claims 1 to 5, **characterised in that** the method is carried out discontinuously.

7. Method according to Claim 6, **characterised in that** the hydrogen pressure is at 3 to 50 bar absolute.

## Revendications

1. Procédé pour la préparation de menthol par hydrogénation catalytique de matières premières, lesquelles possèdent le squelette carboné du menthane avec au moins une double liaison et sont substituées par de l'oxygène en position 3 et/ou par transposition catalytique de stéréoisomères du menthol en présence d'hydrogène, **caractérisé en ce que** l'on réalise la réaction en présence d'un catalyseur de nickel dopé de fer et/ou de chrome et à une température dans le domaine de 80-230°C et à des pression d'hydrogène dans le domaine de 1-200 bars absolu, le catalyseur de nickel dopé présentant à l'état sec une part en fer de 0,1-20 % en poids, une part en chrome de 0,1-20 % en poids, une part en nickel de 60-95 % en poids et une part en aluminium de 1-20 % en poids.

2. Procédé selon la revendication 1, **caractérisé en ce que** le catalyseur de nickel présente à l'état sec une part en fer de 0,1-10 % en poids, une part en chrome de 0,1-10 % en poids, une part en nickel de 80-93 % en poids et une part en aluminium de 3-10 % en poids.

3. Procédé selon au moins l'une quelconque des revendications 1 à 2, **caractérisé en ce que** l'on réalise le procédé essentiellement sans agent diluant.

4. Procédé selon au moins l'une quelconque des revendications 1 à 3, **caractérisé en ce que** le rapport massique du catalyseur de nickel sec aux matières premières se trouve dans le domaine de 0,001-0,1:1.

5. Procédé selon au moins l'une quelconque des revendications 1 à 4, **caractérisé en ce que** la température de réaction est comprise entre 120 et 210°C.

6. Procédé selon au moins l'une quelconque des revendications 1 à 5, **caractérisé en ce que** l'on réalise le procédé en discontinu.

7. Procédé selon la revendication 6, **caractérisé en ce que** la pression d'hydrogène est comprise entre 3 et 50 bars absolu.
